# EUROPEAN PATENT APPLICATION

(11) **EP 2 008 610 A1**
(43) Date of publication of application: **31.12.2008**
(21) Application number: 06833793.0
(22) Date of filing: 30.11.2006
(51) Int. Cl.: A61C 3/02, A61B 18/20

(54) **TREATMENT DEVICE AND TREATMENT METHOD**

(30) Priority: 14.04.2006 JP 2006112100
(71) Applicant: Sumitomo Electric Industries, Ltd., Osaka-shi, Osaka 541-0041 (JP)
(72) Inventor: HATAYAMA, Hitoshi, Yokohama-shi, Kanagawa 244-8588 (JP); INOUE, Akira, Yokohama-shi, Kanagawa 244-8588 (JP); SUGANUMA, Hiroshi, Yokohama-shi, Kanagawa 244-8588 (JP); KATO, Junji, Chiba-shi, Chiba 261-8502 (JP)
(74) Representative: HOFFMANN EITLE
(86) International application number: PCT/JP2006/323990
(87) International publication number: WO 2007/129424

(57) **Abstract**

A dental treatment apparatus is provided with an irradiation unit 10, a temperature detection unit 20 and a control unit 30. The irradiation unit 10 is constituted by N number of first light emitting elements S₁ to S_{N}, N number of first focusing lenses L₁ to L_{N}, and N number of optic fibers F₁ to F_{N}, combined on a 1:1 basis, and optic fibers Fₙ are bundled on the output side of the irradiation unit 10. The first light emitting element Sₙ is an element which outputs laser light having a specific wavelength λ within a range of 400 nm to 420 nm. The temperature detection unit 20 is constituted by a non-contact temperature sensor 22 and a wire 24. The non-contact temperature sensor 22 detects the temperature at a site irradiated with laser light output from an emergent end.

## Description

### Technical Field

The present invention relates to a treatment apparatus and a treatment method.

### Background Art

Treatment apparatuses are required to not only able to treat hard tissue at a treatment site, but also soft tissue at a treatment site. As an example of such an apparatus, the dental treatment apparatus described in Japanese Patent Application Laid-open No. 2004-57658 treats both hard tissue (such as enamel and dentin) and soft tissue (such as gingiva and gums) of teeth using laser light having an oscillation wavelength of 1.5 µm to 4 µm and a pulse width within the range of 250 µs to 1 ms.

### Disclosure of the Invention

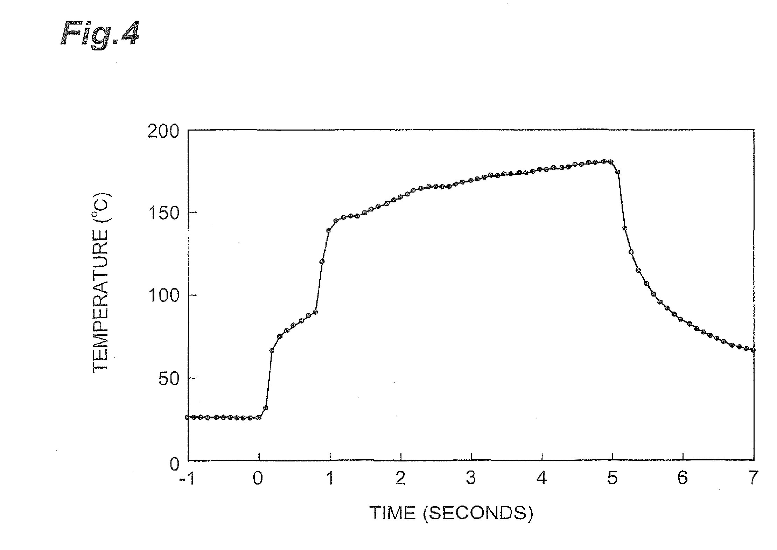

However, since the oscillation wavelength of the laser light used in the above-mentioned dental treatment apparatus is within the range of 1.5 µm to 4 µm, the laser light having this oscillation wavelength of 1.5 µm to 4 µm is easily absorbed by moisture (see FIG. 4). Consequently, it was difficult to obtain satisfactory hemostatic effects when treating dental soft tissue.

In order to solve the above-mentioned problems, an object of the present invention is to provide a treatment apparatus and treatment method, which in addition to being able to treat both hard tissue and soft tissue at a treatment site, allows the obtaining of satisfactory hemostatic effects.

A treatment apparatus according to the present invention is provided with a first light emitting element emitting laser light of a specific wavelength λ₁ within a range of 400 nm to 420 nm, a first focusing lens which focuses laser light output from the first light emitting element, and first output means which outputs laser light output from the first light emitting element and focused by the first focusing lens.

In a treatment apparatus according to the present invention, since laser light output from the light emitting element has a specific wavelength λ₁ within a range of 400 nm to 420 nm, energy of the laser light is effectively absorbed by protein contained in hard tissue at the treatment site, is instantaneously transformed to thermal energy, and transpirates the irradiated site. In addition, since laser light of 400 nm to 420 nm is effectively absorbed by hemoglobin and myoglobin in soft tissue, it is also possible to transpirate soft tissue at a treatment site. In this manner, this treatment apparatus is able to treat both soft tissue and hard tissue at a treatment site. In addition, since the wavelength region of the laser light is within the range of 400 nm to 420 nm, the water absorption coefficient is low and tissue penetrability is greater than intermediate and far infrared light, thereby enabling the formation of a suitable denatured layer around the irradiated site and anticipation of hemostatic effects.

In a treatment apparatus according to the present invention, the first output means is preferably an optic fiber which inputs and guides laser light focused by the first focusing lens to an incident end and outputs the light from an emergent end. In this case, together with this facilitating the propagation of laser light, transmission loss occurring during guiding of the laser light can be suppressed.

In a treatment apparatus according to the present invention, the numerical aperture at the light output end of the first output means is preferably less than 0.14. In this case, effects causing transpiration of the treatment site can be enhanced, and incision efficiency can be improved.

A treatment apparatus according to the present invention is provided with (1) N number of first light emitting elements S₁ to S_{N} respectively outputting laser light of a specific wavelength λ₁, (2) N number of first focusing lenses L₁ to L_{N} provided on a 1:1 basis for the N number of light emitting elements S₁ to S_{N} which focus laser light output from each corresponding first light emitting element, and (3) N number of optic fibers F₁ to F_{N}, which input and guide laser light output from the N number of first light emitting elements S₁ to S_{N} and focused by the N number of first focusing lenses L₁ to L_{N}, to an incident end, and output the light from an emergent end; wherein, n number of the optic fibers Fₙ are bundled at the emergent end (wherein, N represents an integer of 2 or more, and n represents any arbitrary integer from 1 to N).

Since a treatment apparatus according to the present invention is respectively provided with first light emitting elements Sₙ and first focusing lenses Lₙ on a 1:1 basis for the optic fibers Fₙ, the efficiency of photocoupling from the first light emitting elements Sₙ to the incident ends of the optic fibers Fₙ can be enhanced. Laser light output from each first light emitting element Sₙ is focused by a corresponding first light focusing lens Lₙ, enters the incident end of a corresponding optic fiber Fₙ, is guided by that optic fiber Fₙ, and is efficiently output to the outside from the emergent end of that optic fiber Fₙ. Moreover, since the emergent ends of the optic fibers Fₙ are bundled, laser light output from the first light emitting elements Sₙ is gathered, enabling the obtaining of laser light having a high power density.

A treatment apparatus according to the present invention is preferably provided with a control unit which controls the power of laser light output from the first light emitting elements Sₙ. In this case, the power of the laser light can be adjusted corresponding to the treatment requirements, and the performance and applicability of the apparatus can be improved.

A treatment apparatus according to the present invention is preferably installed with a non-contact temperature sensor on an emergent end of the optic fiber Fₙ which detects the temperature of a site irradiated with laser light output from that emergent end, and the control unit preferably controls the power of the laser light output from the first light emitting elements Sₙ based on the temperature result detected with the non-contact temperature sensor. In this case, damage to tissue at locations other than the irradiated site can be suppressed by precisely managing the temperature rise at the irradiated site and controlling the power of the output laser light.

A treatment apparatus according to the present invention preferably controls the power of the laser light output from the first light emitting elements Sₙ so as to change the irradiation intensity of the laser light output from the first light emitting elements Sₙ over time. In this case, damage to tissue at locations other than the irradiated site can be suppressed by controlling the power of the output laser light.

A treatment apparatus according to the present invention is preferably provided with a second light emitting element which emits laser light of a specific wavelength λ₂ within a range of 800 nm to 1000 nm, a second focusing lens which focuses laser light output from the second light emitting element, and second output means which outputs laser light output from the second light emitting element and focused by the second focusing lens.

Since laser light of a specific wavelength λ₂ within the range of 800 nm to 1000 nm has high tissue penetrability, a suitable denatured layer can be formed around the irradiated site, enabling anticipation of hemostatic effects.

In a treatment apparatus according to the present invention, a control unit preferably controls the power of laser light output from the second light emitting element so as to change the irradiation intensity of the laser light output from the second light emitting element over time. In this case, damage to tissue at locations other than the irradiated site can be suppressed by controlling the power of the output laser light.

In a treatment apparatus according to the present invention, the first output means and second output means preferably each radiate laser light so that the respective output laser light is mutually and spatially overlapping. In this case, treatment can be performed more effectively by combining the use of laser light of a specific wavelength λ₁ and a specific wavelength λ₂.

In a treatment apparatus according to the present invention, at an irradiated spot formed by the respective laser light from the first output means and the second output means, laser light of a mutually different wavelength at the vicinity of the center of the irradiated spot and at the periphery thereof is preferably radiated by the first output means and the second output means. In this case, treatment can be performer more effectively by facilitating the handling of laser light of two types of specific wavelengths.

In a treatment apparatus according to the present invention, laser light is preferably irradiated at the periphery of the irradiated spot by the first output means. In this case, laser light of a specific wavelength λ₁ can be first irradiated at a treatment site along the scanning direction of the laser light, and laser light of a specific wavelength λ₂ can be irradiated subsequent thereto, thereby allowing the obtaining of satisfactory transpiration effects and hemostatic effects.

A treatment method according to the present invention treats at least hard tissue or soft tissue at a treatment site by irradiating with laser light having a specific wavelength of 400 nm to 420 nm.

In a treatment method according to the present invention, since laser light is effectively absorbed by protein contained in hard tissue at a treatment site when laser light having a specific wavelength of 400 nm to 420 nm is radiated onto hard tissue at the treatment site, the energy of the absorbed laser light is instantaneously transformed into thermal energy, and a result of transpiration of the hard tissue, the treatment site can be treated. In addition, since laser light is effectively absorbed by hemoglobin and myoglobin when this laser light having a specific wavelength of 400 nm to 420 nm is radiated on to soft tissue at a treatment site, the soft tissue is transpirated enabling an incision to be made therein. Moreover, since only a small amount of this laser light is absorbed by water and has comparatively high tissue penetrability, a denatured layer of a suitable thickness can be formed, resulting in the obtaining of satisfactory hemostatic effects.

A treatment method according to the present invention treats at least hard tissue or soft tissue at a treatment site by irradiating with laser light having a specific wavelength of 400 nm to 420 nm and laser light having a specific wavelength of 800 nm to 1000 nm.

In a treatment method according to the present invention, since laser light having a specific wavelength of 800 nm to 1000 nm has hemostatic effects on a treatment site, the combined use of laser light of both specific wavelengths makes it possible to perform treatment more effectively.

In addition, in a treatment method according to the present invention, irradiation with the laser light having a specific wavelength of 400 nm to 420 nm is preferably carried out by changing the irradiation intensity over time based on the temperature of a treatment site. In this case, by adjusting the irradiation intensity of the laser light in conformity with the treatment site, the formation of cracks and carbonization at the treatment site, which occur easily in cases of continuous irradiation, can be suppressed, while also enabling damage to normal tissue at locations other than the treatment site to be suppressed.

In addition, in a treatment method according to the present invention, in the case of irradiation with at least one of the laser light having a specific wavelength of 400 nm to 420 nm and the laser light having a specific wavelength of 800 nm to 1000 nm, it is preferable to change the irradiation intensity over time based on the temperature of the treatment site. In this case, by adjusting the irradiation intensity of the laser light in conformity with the treatment site, the formation of cracks and carbonization at the treatment site, which occur easily in cases of continuous irradiation, can be suppressed, while also enabling damage to normal tissue at locations other than the treatment site to be suppressed.

### Advantage

According to the present invention, in addition to being able to treat both hard tissue and soft tissue at a treatment site, the treatment apparatus and treatment method of the present invention also allows the obtaining of satisfactory hemostatic effects.

### Brief Description of the Drawings

FIG. 1 a block drawing of a dental treatment apparatus as claimed in a first embodiment.
Fig. 2 is a drawing shown the optical absorption spectrum of dentin.
FIG. 3 is a drawing showing the absorption spectrum of water.
FIG. 4 is a drawing showing the relationship between temperature rise and time of bovine dentin.
FIG. 5 is a drawing showing pulsed irradiation of laser light.
FIG. 6 is a drawing showing the irradiation conditions of Examples 1 to 4.
FIG. 7 is a drawing showing the irradiation results of Examples 5 to 7.
FIG. 8 is a block drawing of a treatment apparatus as claimed in a second embodiment.
FIG. 9 is a drawing showing the optical absorption spectrum of oxygenated hemoglobin.
FIG. 10 is a drawing showing the irradiation conditions of Examples 8 to 10 and Comparative Example 11.
FIG. 11 is a drawing showing the irradiation results of Examples 8 to 10 and Comparative Example 11.
FIG. 12 is a drawing showing the constitution of an irradiated spot.
FIG. 13A, B and C are drawings showing variations of irradiated spots, respectively.

### Description of Reference Symbols

- 1: dental treatment apparatus (treatment apparatus)
- 2: treatment apparatus
- 10: irradiation unit
- 20: temperature detection unit
- 22: non-contact temperature sensor
- 30: control unit
- 42: first light emitting element
- 44: first focusing lens
- 46: first optic fibers (first output means)
- 52: second light emitting element
- 54: second focusing lens
- 56: second optic fibers (second output means)
- 60: irradiated spot,
- S₁-S_{N}: first light emitting element
- L₁-L_{N}: first focusing lens
- F₁-F_{N}: optic fiber

### Best Mode for Carrying out the Invention

The following provides a detailed explanation of embodiments of the present invention with reference to the attached drawings. Furthermore, the same reference symbols are indicated for the same constituent elements in the explanation of the drawings, and duplicate explanations are omitted.

### (First Embodiment)

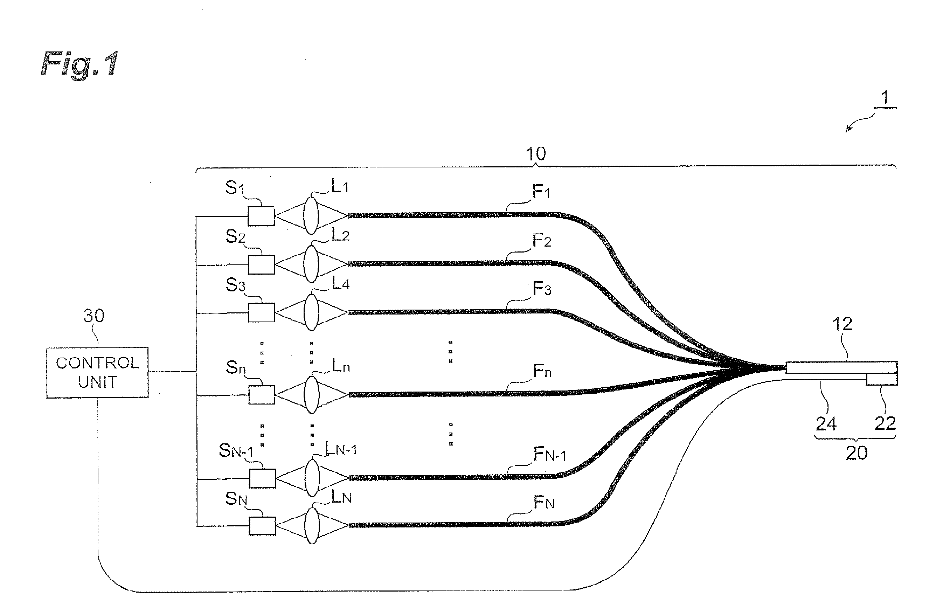

The following provides an explanation of a treatment apparatus and treatment method as claimed in the present embodiment using the example of a dental treatment apparatus. Furthermore, the treatment apparatus as claimed in the present invention is not limited to a dental treatment apparatus. FIG. 1 is a block drawing of a dental treatment apparatus 1 as claimed in the first embodiment. As shown in this drawing, the dental treatment apparatus 1 is provided with an irradiation unit 10, a temperature detection unit 20 and a control unit 30.

The irradiation unit 10 is constituted by N number of first light emitting elements S₁ to S_{N}, N number of first focusing lenses L₁ to L_{N}, and N number of optic fibers (first output means) F₁ to F_{N} combined on a 1:1 basis. Here, N is an integer of 2 or more, and n to be appear later is an arbitrary integer of 1 to N. The first light emitting elements S₁ to S_{N}, the first focusing lenses L₁ to L_{N} and the optic fibers F₁ to F_{N} are preferably each the same. In addition, optic fibers Fₙ are bundled and held by a ferrule 12 on the output side of the dental treatment apparatus 1.

A first light emitting element Sₙ is an element which emits laser light at a specific wavelength λ₁ within the range of 400 nm to 420 nm, and preferably includes a semiconductor laser element which outputs laser light having a specific wavelength λ₁ within the range of 400 nm to 420 nm. A first focusing lens Lₙ is that in which both lenses are convex lenses, is arranged between the first light emitting element Sₙ and an optic fiber Fₙ, focuses light output from the first light emitting element Sₙ and causes said light to enter the incident end of the optic fiber Fₙ. The optic fiber Fₙ is provided on a 1:1 basis with respect to the first light emitting element Sₙ and the first focusing lens Lₙ, the incident end of the optic fiber Fₙ is optically connected to the first light emitting element Sₙ via the first focusing lens Lₙ, and the emergent end is bundled and held with the ferrule 12.

The temperature detection unit 20 is constituted by a non-contact temperature sensor 22 and a wire 24. More specifically, the non-contact temperature sensor 22 is attached to the emergent end of the optic fiber Fₙ of the irradiation unit 10, and detects in a non-contact manner far infrared light emitted from a site (irradiated site) irradiated with laser light output from the emergent end. In addition, the non-contact temperature sensor 22 is connected to the control unit 30 via the wire 24. Thus, temperature data detected with the non-contact temperature sensor 22 is transmitted to the control unit 30 through the wire 24.

The control unit 30 is provided on the side of the first light emitting element Sₙ of the dental treatment apparatus 1, and control the power of light output from each of the N number of the first light emitting elements S₁ to S_{N}. Namely, the control unit 30 adjusts the on/off time of laser light output from, for example, the first light emitting element Sₙ based on the temperature result of an irradiated site detected with the non-contact temperature sensor 22 and transmitted by the wire 24, and controls the power of output laser light by changing the irradiation intensity of the laser light output from the first light emitting element Sₙ over time.

Here, an explanation is provided of treating both dental hard tissue and soft tissue with laser light having a specific wavelength of 400 nm to 420 nm used in the dental treatment apparatus 1.

Dental hard tissue is composed of dentin, enamel and cementum, and the majority is composed of dentin and enamel. Dentin is the main component of dental hard tissue, and its components consist of 69% inorganic matter, 18% organic matter and 13% water. On the other hand, enamel is composed of 96% inorganic matter, 2% organic matter and 2% water.

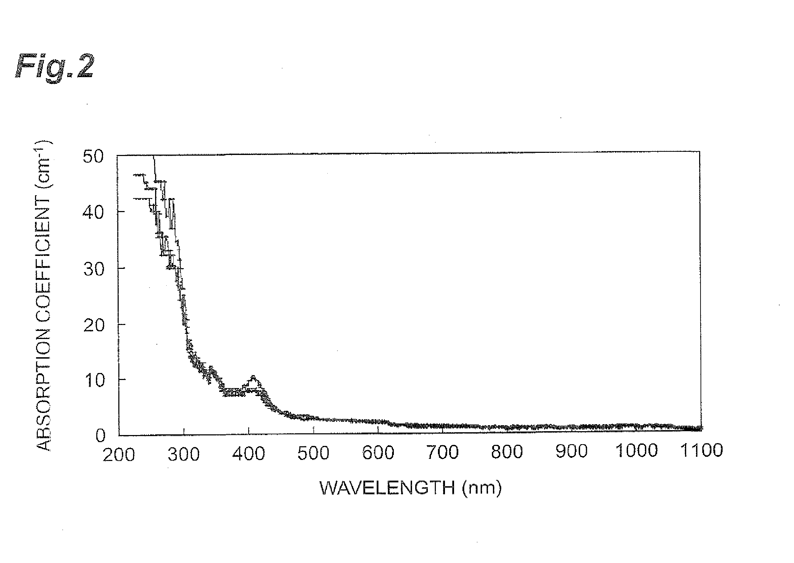

FIG. 2 shows an optical absorption spectrum of dentin (thickness: 900 µm). As shown in this drawing, the absorption coefficient of dentin tends to increase as the wavelength becomes shorter. In terms of medical treatment, light of a wavelength shorter than 400 nm (namely, ultraviolet light and the like) is said to have the risk of destroying DNA of the body, and it is therefore not desirable to use light having a wavelength shorter than 400 nm in treatment. Since the laser light of the dental treatment apparatus 1 as claimed in the present embodiment is within a wavelength range of 400 nm to 420 nm, it has little detrimental effects on the body. In addition, as shown in FIG. 2, among wavelengths longer than 400 nm, dentin has an optical absorption peak in the vicinity of a wavelength of 405 nm. Consequently, laser light is effectively absorbed, is instantaneously transformed into thermal energy, and is capable of causing transpiration of an irradiated site (namely, a treatment site). As a result, in the case a cavity or other lesion is present at the irradiated site, that lesion can be removed thereby resulting in therapeutic effects.

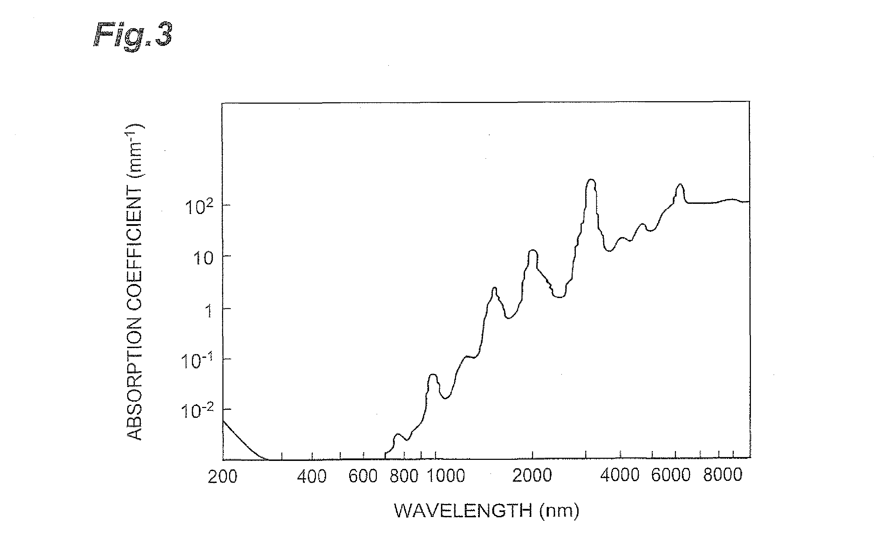

On the other hand, 80% of the tissue components of dental soft tissue (such as the gingiva) is water. In order to incise soft tissue with laser light while effectively maintaining hemostasis, laser light having a wavelength at which absorption by water is to a certain extent low while having a certain extent of tissue penetrability is suitable. As shown in FIG. 3, the laser light having a specific wavelength of 400 nm to 420 nm used in the dental treatment apparatus 1 demonstrates low absorption by water. On the other hand, it is absorbed well by hemoglobin and myoglobin, and has suitable tissue penetrability. As a result, the laser-irradiated site can be incised while maintaining effective hemostasis.

The following provides an explanation of a dental treatment method using the above-mentioned dental treatment apparatus 1.

First, an explanation is provided of cutting dentin in the case of treating dental hard tissue. A dentin treatment site is irradiated with laser light having a specific wavelength of 400 nm to 420 nm using the dental treatment apparatus 1. At this time, the energy of the laser light is absorbed by protein in the dentin and is instantaneously transformed into thermal energy resulting in transpiration of the dentin. As a result, the dentin can be cut. Furthermore, it is preferable to intermittently irradiate the treatment site with the laser light so as to prevent the occurrence of cracks or carbonization in the tooth caused by the high level of heat at this time. Namely, the dentin of teeth is susceptible to the occurrence of cracking and carbonization by heat in the case of continuous irradiation of the dentin beyond that which is necessary. In order to prevent the occurrence thereof, it is preferable to adjust the on/off time of laser light output from, for example, the first laser emitting element Sₙ, or change the emergent intensity of laser light output from the first light emitting element Sₙ over time.

FIG. 4 is a drawing showing the relationship between time and temperature rise when irradiating bovine dentin for 5 seconds with laser light at a wavelength of 405 nm under conditions of a laser light irradiation power of 1.7 W, power density of 30 W/mm², irradiation beam diameter of 270 µm, room temperature of 30°C and location of the temperature measurement point being at a location 600 µm away from the irradiation center. The horizontal axis indicates time based on the irradiation starting time, while the vertical axis indicates temperature. As shown in this drawing, within the range from the start of irradiation to within 0.2 seconds from the start thereof, the temperature of the dentin rose rapidly, and within the range of 0.2 seconds to 0.8 seconds, the temperature rise became gradual. Within the range of 0.8 seconds to 1 second after the start of irradiation, the temperature of the dentin again rose rapidly, with the temperature of the dentin reaching 120°C at 0.9 seconds after the start of irradiation, at which time the occurrence of drilling into the dentin was confirmed. Moreover, the temperature of the dentin gradually continued to rise within the range of 1 second to 5 seconds after the start of irradiation, cracks and carbonization occurred in the dentin, and the carbonized area gradually increased, with the temperature of the dentin at 5 seconds after the start of irradiation reaching 180°C.

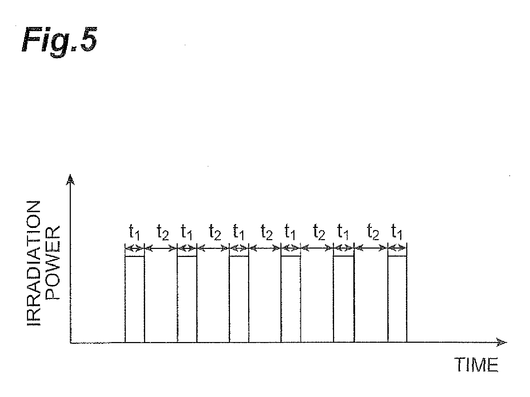

On the basis of the above results, the occurrence of cracking and carbonization at a treatment site during treatment of dentin is suppressed by a pulsed irradiation treatment method in which the irradiation on time t₁ (t₁ = 0.9 seconds) and the irradiation off time t₂ (t₂ = 2 seconds) are repeated at a fixed cycle to intermittently change the irradiation intensity as shown in FIG. 5. In addition, in the case of this type of pulsed irradiation treatment, the treatment site may be cooled with water or cooling air during the irradiation off time t₂. This enables treatment to be performed more efficiently.

In addition, it is preferable to use the non-contact temperature sensor 22 of the temperature detection unit 20 to manage the temperature of the treatment site by detecting far infrared rays emitted from the treatment site with the non-contact temperature sensor 22. More specifically, far infrared rays emitted from the treatment site are detected with the non-contact temperature sensor 22, and the detected results are transmitted to the control unit 30 via the wire 24. The control unit 30 then adjusts the on/off time of the laser light output from, for example, the first light emitting element Sₙ based on the transmitted temperature results, and changes the emergent intensity of the laser light output from the first light emitting element Sₙ over time to control the average power of the output laser light. As a result, the temperature rise at the treatment site can be controlled more precisely, thereby making it possible to realize treatment with little damage to normal tissue. Furthermore, even in the case of the pulsed irradiation described above, it is not necessary to irradiate intermittently using fixed values for the irradiation on time t₁ and the irradiation off time t₂, but rather t₁ and t₂ may be suitably changed corresponding to the status (e.g., temperature) of the treatment site.

Similarly, when treating dental soft tissue as during, for example, incision into the gingiva, for example, an irradiated site of the gingiva is irradiated with laser light within the wavelength range of 400 nm to 420 nm sing the dental treatment apparatus 1. Since laser light in this wavelength range has high penetrability of soft tissue to a certain extent, in addition to the irradiated site being transpirated and incised as a result of irradiating with the laser light, a denatured layer of a suitable thickness is formed, thereby allowing the obtaining of satisfactory hemostatic effects.

According to the present embodiment, since the dental treatment apparatus 1 is provided with light emitting elements emitting laser light having a specific wavelength of 400 nm to 420 nm, and laser light within this wavelength range of 400 nm to 420 nm is effectively absorbed by protein in dentin, thereby demonstrating suitable penetrability into soft tissue, it is able to treat both dental hard tissue and soft tissue.

In addition, since the dental treatment apparatus 1 is provided with a first focusing lens Lₙ and an optic fiber Fₙ corresponding to a first light emitting element Sₙ on a 1:1 basis, the efficiency of photocoupling from the first light emitting element Sₙ to the incident end of the optic fiber Fₙ can be increased. Consequently, laser light output from the first light emitting element Sₙ is focused by the corresponding first focusing lens Lₙ, enters the incident end of the corresponding optic fiber Fₙ, is guided by the optic fiber Fₙ, and is efficiently emitted to the outside from the emergent end of the optic fiber Fₙ.

In addition, since the optic fiber Fₙ is bundled at the emergent end thereof, laser light output from the first light emitting element Sₙ is focused, the arrangement density of the optic fiber Fₙ at the emergent end increases, and laser light of a high power density can be obtained, thereby making it possible to anticipate effects which improve the performance and applicability of the dental treatment apparatus 1. The use of the optic fiber Fₙ not only facilitates propagation of light, but also makes it possible to suppress transmission loss occurring when the light is guided by the optic fiber Fₙ. In addition, as a result of the optic fiber Fₙ being bundled at the emergent end thereof, workability during production of the dental treatment apparatus 1 can be improved and handling of the apparatus during treatment becomes easy. Moreover, by using an inexpensive and compact semiconductor light emitting element for the first light emitting element Sₙ, the size and cost of the dental treatment apparatus 1 can be reduced.

The following provides a more detailed explanation of the first embodiment using examples thereof.

### Examples 1-4

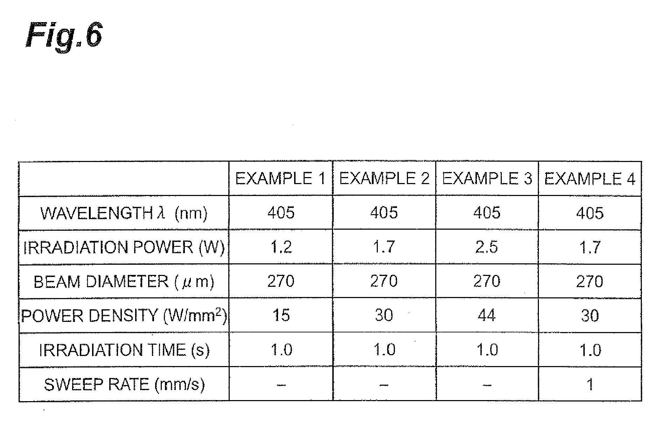

First, as shown in FIG. 6, bovine dentin was irradiated to cut the dentin using laser light under four sets of irradiation conditions comprised of parameters consisting of the wavelength of the laser light, irradiation power, beam diameter, power density, irradiation time and sweep rate.

As a result, although there were no changes observed in the appearance of the dentin attributable to irradiation under the conditions of Example 1, the dentin was confirmed to have been cut under the irradiation conditions of Examples 2 to 4.

According to the above results, laser light irradiated under the conditions of Examples 2 to 4 was verified to have the effect of being able to treat dental hard tissue. Under the irradiation conditions of Example 1, the power density of the laser light was excessively low, thus making these conditions inadequate for treatment.

### Examples 5-7

In addition, soft tissue was cut under irradiation conditions of a laser light wavelength of 405 nm, power of 1.7 W, beam diameter of 270 µm, power density of 30 W/mm², irradiation time of 1.0 second, and sweep rate of 1.0 mm/s using tuna as a typical example of soft tissue containing a large amount of lipids in Example 5, chicken white meat as a typical example of muscle tissue in Example 6, and liver as a typical example of tissue containing a large amount of hemoglobin in Example 7.

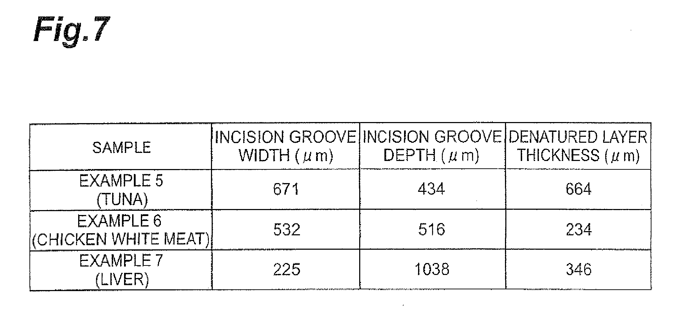

As a result, incisions into the samples were able to be confirmed in Examples 5 to 7 as shown in FIG. 7. In addition, denatured layers of a suitable thickness were formed in all the samples, thus making it possible to expect incisions with satisfactory hemostasis. Furthermore, although experiment results are shown for non-human teeth and tissues in the above-mentioned examples, it is clear that results similar to those described above can be obtained in the case of human teeth and gingiva as well.

### (Second Embodiment)

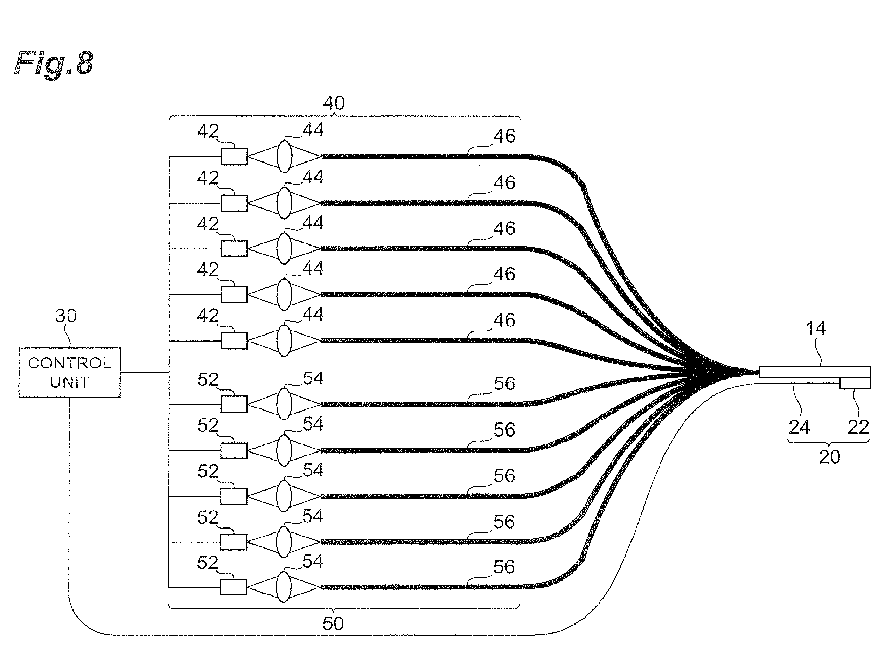

Next, an explanation is provided of a second embodiment of a treatment apparatus as claimed in the present embodiment. FIG. 8 is a block drawing of a treatment apparatus as claimed in the second embodiment. A treatment apparatus 2 is provided with a first irradiation unit 40, a second irradiation unit 50, a temperature detection unit 20, and a control unit 30.

The first irradiation unit 40 is constituted by (1) five first light emitting elements 42, which emit laser light having a specific wavelength λ₁ within the range of 400 nm to 420 nm, (2) five first focusing lenses 44, which focus laser light output from the first light emitting elements 42, and (3) five first optic fibers (first output means) 46, which guide laser light output from the first light emitting elements 42 and focused by the first focusing lenses 44, input the laser light to incident ends thereof and output the light from emergent ends thereof, respectively combined on a 1:1 basis.

The second irradiation unit 50 is constituted by (1) five second light emitting elements 52, which emit laser light having a specific wavelength λ₂ within the range of 800 nm to 1000 nm, (2) five second focusing lenses 54, which focus laser light output from the second light emitting elements 52, and (3) five second optic fibers (second output means) 56, which guide laser light output from the second light emitting elements 52 and focused by the second focusing lenses 54, input the laser light to incident ends thereof and output the light from emergent ends thereof, respectively combined on a 1:1 basis.

The first light emitting elements 42 and the second light emitting elements 52 are elements that emit light, and preferably include semiconductor laser elements that output laser light. The first focusing lenses 44 and the second focusing lenses 54 are lenses in which both sides are convex, and focus laser light having a specific wavelength λ₁ emitted from the first light emitting elements 42 and light having a specific wavelength λ₂ emitted from the second light emitting elements 52, respectively. The first optic fibers 46 and the second optic fibers 56 are bundled at one end thereof (emergent end), are held by a ferrule 14, and the other ends are optically connected to the first light emitting elements 42 and the second light emitting elements 52 via the first focusing lenses 44 and the second focusing lenses 54, respectively.

In the treatment apparatus 2, the numerical aperture at each emergent end of the first optic fibers 46 and the second optic fibers 56 is preferably less than 0.14. In this case, since the laser light can be focused on a small area, the effect of transpirating a treatment site can be enhanced, and incision efficiency can be improved. Furthermore, although examples of irradiating tissue directly with light output from optic fibers are explained in the aforementioned first and second embodiments, lenses and so on may also be installed on the output ends of these optic fibers as necessary to adjust the laser light. In this case, the numerical aperture at the output ends of the light of the above-mentioned output means refers to the numerical aperture at the output end of the light of the output means immediately prior to irradiating tissue.

Since the structure, installation and so on of the temperature detection unit 20 are the same as the above-mentioned first embodiment, a duplicate explanation thereof is omitted. The control unit 30 is provided on the side of the first light emitting elements 42 and the second light emitting elements 52 of the treatment apparatus 2, and controls the power of laser light emitted from these light emitting elements based on temperature results at the irradiated site detected with a non-contact temperature sensor 22 and transmitted by a wire 24 so as to change the irradiation intensity of the respective laser light output from the first light emitting elements 42 and the second light emitting elements 52 over time.

Here, an explanation is provided of treatment of a treatment site with laser light having a specific wavelength λ₁ within the range of 400 nm to 420 nm and laser light having a specific wavelength λ₂ within the range of 800 nm to 1000 nm used in the treatment apparatus 2.

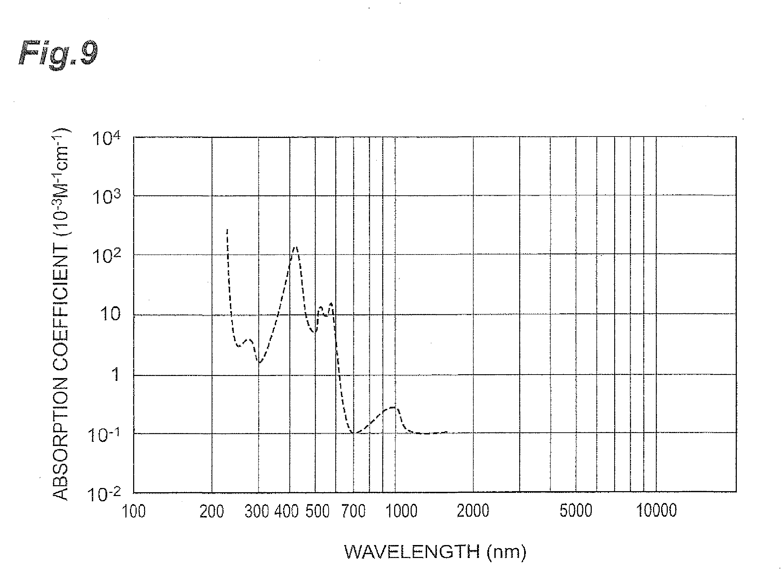

FIG. 9 is a drawing showing the optical absorption spectrum of oxygenated hemoglobin. As shown in this drawing, although the absorption coefficient of oxygenated hemoglobin tends to increase the shorter the wavelength, as was previously described, since light having a wavelength shorter than 400 nm is said to have the risk of destroying DNA of the body, and it is therefore not desirable to use light having a wavelength shorter than 400 nm in treatment. In addition, as shown in FIG. 9, among light having a wavelength longer than 400 nm, light having a wavelength of 400 nm to 420 nm is effectively absorbed by oxygenated hemoglobin.

Thus, when irradiating a treatment site with laser light having a wavelength of 400 nm to 420 nm, the laser light of that frequency is absorbed by oxygenated hemoglobin contained in large amounts in the blood of soft tissue and is instantaneously transformed into heat, thereby enabling the soft tissue to be efficiently transpirated. As a result, the effect is obtained that allows soft tissue to be efficiently incised. In addition, since laser light of the wavelength indicated above is effectively absorbed by oxygenated hemoglobin, the blood can be made to coagulate easily, thereby enhancing hemostatic effects.

In addition, since laser light having a specific wavelength λ₂ within the range of 800 nm to 1000 nm demonstrates a high tissue permeation rate, a denatured layer of a suitable thickness can be formed around the irradiated site, thereby also enhancing hemostatic effects.

The following provides an explanation of examples of using laser light having a specific wavelength λ₁ within the range of 400 nm to 420 nm.

### Examples 8-10 and Comparative Example

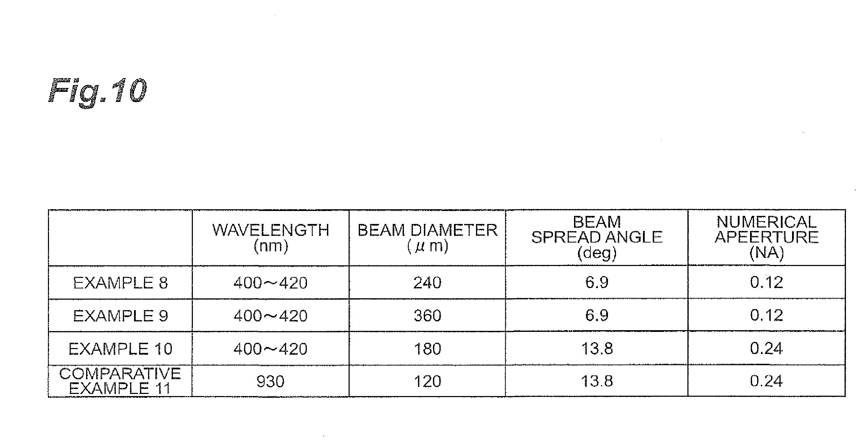

As shown in FIG. 10, tissue transpiration depth was measured by irradiating with laser light under four sets of irradiation conditions constituted by the parameters consisting of laser light wavelength, beam diameter, beam spread angle and numerical aperture at the laser light output end using tuna soft tissue containing a large amount of myoglobin demonstrating similar absorption characteristics to those of oxygenated hemoglobin. In addition, the tissue was also irradiated with semiconductor laser light having a wavelength of 930 nm for comparison. Furthermore, irradiation was carried out by aligning the focal point of the laser light with surface of the tuna soft tissue, and irradiating under conditions of a scanning rate of 1 mm/s.

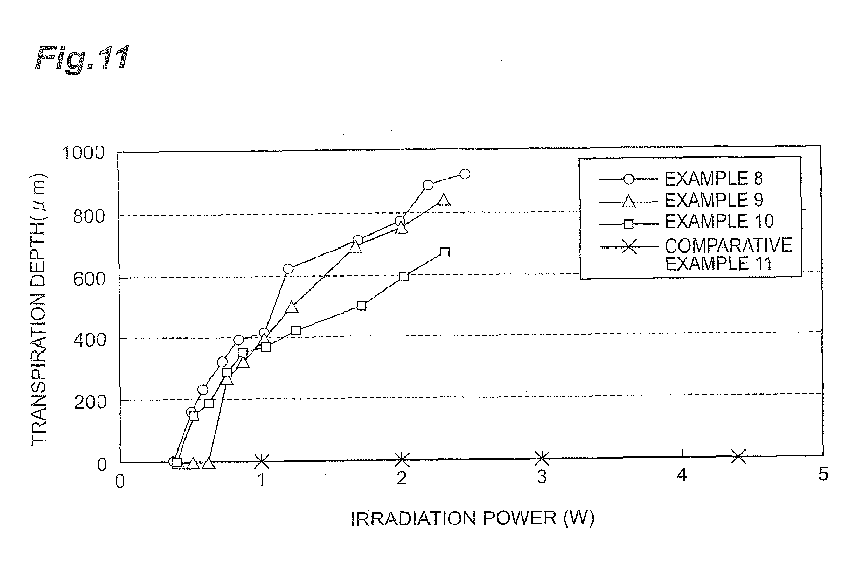

As a result, as shown in FIG. 11, tissue transpiration was not observed in the case of irradiating with laser light having a wavelength of 930 nm (see Example 11), while tissue was observed to be efficiently transpirated by laser light at 400 nm to 420 nm (see Examples 8 to 10). Thus, laser light having a wavelength of 400 nm to 420 nm was demonstrated to have effects that enable tissue to be efficiently incised.

In addition, better incisability is obtained in the case of laser light having a wavelength of 400 nm to 420 nm with a narrow beam diameter and narrow beam spread angle. This is because, the threshold value of optical output power required for tissue transpiration can be lowered since a smaller beam diameter makes it possible to increase the optical power density of the irradiated portion, and since a narrower beam spread angle makes it possible locally irradiated to a greater depth, power density can be maintained at a high level even at locations deep below the tissue surface, thereby enabling tissue to be incised more efficiently.

Next, an explanation is provided for a treatment method using the treatment apparatus 2.

First, a treatment site is irradiated with laser light having a specific wavelength λ₁ and laser light having a specific wavelength λ₂ using the treatment apparatus 2. At this time, the treatment site is preferably irradiated first along the scanning direction of the laser light with the laser light having a specific wavelength λ₁, followed by irradiating with the laser light having a specific wavelength λ₂.

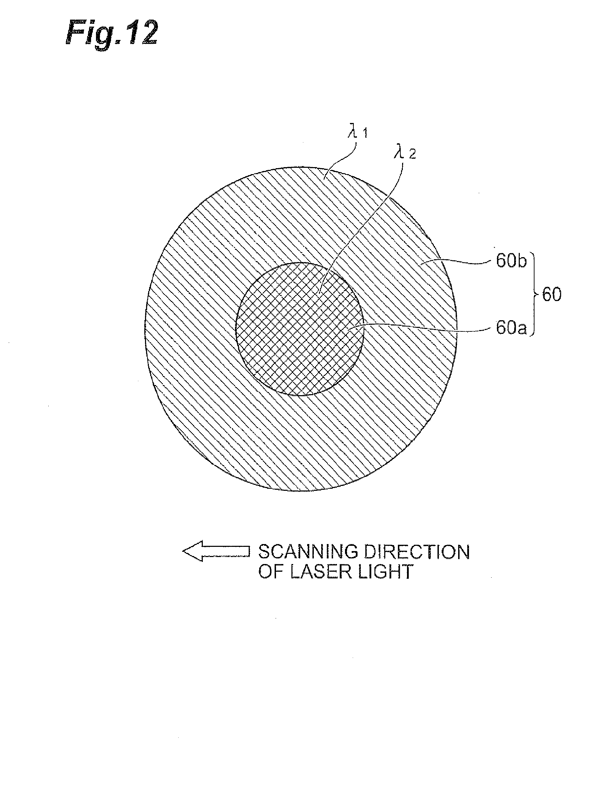

Namely, as shown in FIG. 12, the tissue is irradiated with laser light having mutually different wavelengths at a center 60a and a periphery 60b of an irradiated spot 60 formed by laser light output from the first optic fibers 46 and the second optic fibers 56. More specifically, laser light of a specific wavelength λ₂ is irradiated at the center 60a of the irradiated spot 60, while laser light having a specific wavelength λ₁ is irradiated at the periphery 60b serving as an outer border of the irradiated spot 60.

When irradiating the treatment site while scanning with the laser light using the irradiated spot 60 formed in this manner, the treatment site is irradiated with laser light having a wavelength of 400 nm to 420 nm of the periphery 60b of the irradiated spot 60. Consequently, the treatment site is transpirated and incised. As a result of continuing to scan the irradiated spot 60, the treatment site is irradiated with laser light having a wavelength of 800 nm to 1000 nm of the center 60a of the irradiated spot 60. Since laser light having a wavelength of 800 nm to 1000 nm demonstrates a high tissue permeation rate, it demonstrates hemostatic effects on an incised treatment site. Since the treatment site is first irradiated with light having a wavelength of 400 nm to 420 nm in particular, the permeation rate of infrared light of the tissue decreases, while the absorption rate of near infrared light in the wavelength range of 800 nm to 1000 nm in the vicinity of the incision increases. As a result of then irradiating the treatment site previously irradiated with laser light at 400 nm to 420 nm with laser light having a specific wavelength λ₂, the laser light having a specific wavelength λ₂ is absorbed more effectively, thereby further enhancing hemostatic effects at the treatment site.

In this manner, by combining the use of laser light having a specific wavelength λ₁ and laser light having a specific wavelength λ₂, better treatment site incisability and hemostatic effects can be simultaneously obtained than in the case of irradiating with either laser light alone. Furthermore, when irradiating a treatment site, laser light having a specific wavelength λ₁ and laser light having a specific wavelength λ₂ may be irradiated simultaneously or alternately.

In addition, when irradiating with the laser light, the power of the laser light output from the first light emitting elements 42 and the second light emitting elements 52 is preferably respectively controlled by means of the control unit 30 based on the temperature at the treatment site detected by the temperature detection unit 20 in the same manner as in the first embodiment, or the irradiation intensity of the laser light output from these light emitting elements is preferably changed over time. In this case, by adjusting the irradiation intensity of the laser light in conformity with the treatment site, the occurrence of cracks and carbonization at the treatment site, which occur easily in the case of continuous irradiation, can be suppressed while also enabling damage to normal tissue at locations other than the treatment site to be suppressed.

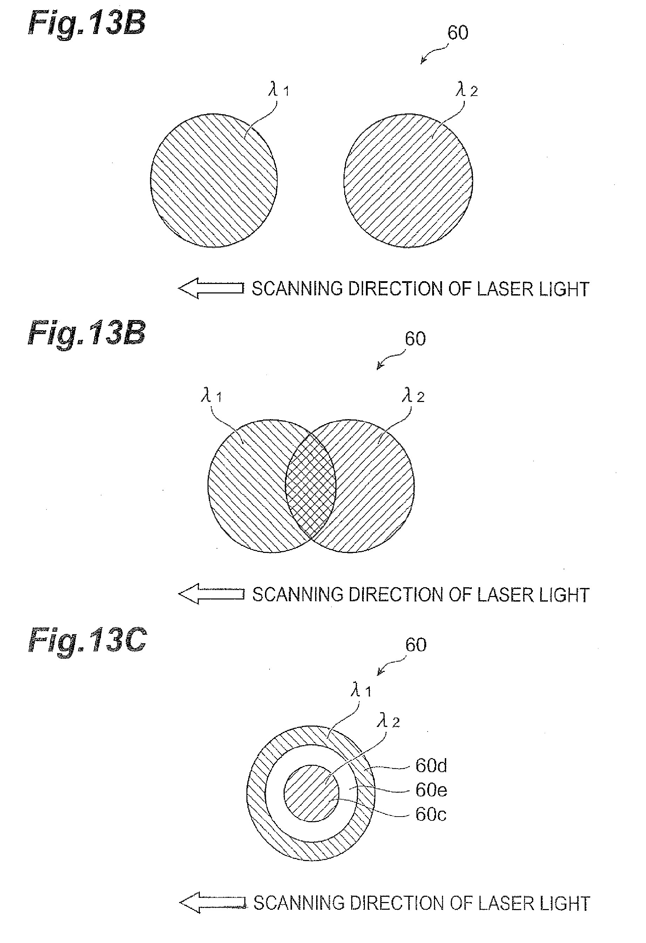

The following provides an explanation of variations of the irradiated spot 60 formed by laser light output from the first optic fibers 46 and the second optic fibers 56 with reference to FIGS. 13A to 13C. In the variation shown in FIG. 13A, laser light having a specific wavelength λ₁ output from the first optic fibers 46 and laser light having a specific wavelength λ₂ output from the second optic fibers 56 are arranged such that the laser light having a specific wavelength λ₁ is used to first irradiate the treatment site followed by irradiating the treatment site with the laser light having a specific wavelength λ₂ along the scanning direction indicated by the arrow in the state in which portions thereof are not mutually and spatially overlapping.

In the variation shown in FIG. 13B, laser light having a specific wavelength λ₁ output from the first optic fibers 46 and laser light having a specific wavelength λ₂ output from the second optic fibers 56 are arranged such. that the laser light having a specific wavelength λ₁ is used to first irradiate the treatment site followed by irradiating the treatment site with the laser light having a specific wavelength λ₂ along the scanning direction indicated by the arrow in the state in which portions thereof are mutually and spatially overlapping.

In the variation shown in FIG. 13C, a center 60c of the irradiated spot 60 is irradiated with laser light having a specific wavelength λ₂ output from the second optic fibers 56, while a periphery 60d thereof is irradiated with laser light having a specific wavelength λ₁ output from the first optic fibers 46. The center 60c and the periphery 60d are separated by a blank area formed in the shape of a ring which is not irradiated by either laser light. Naturally, the above-mentioned blank area is not required to be present, but rather portions of the area irradiated with laser light having a specific wavelength λ₂ and the area irradiated with laser light having a specific wavelength λ₁ may also be overlapping.

Furthermore, the present invention is not limited to the above-mentioned embodiments. For example, although the explanation of the first embodiment used the example of a dental treatment apparatus 1 for the treatment apparatus, the treatment apparatus is not limited to the dental treatment apparatus 1, but rather can also be used as a semiconductor laser scalpel or other treatment apparatus.

### Industrial Applicability

According to the present invention, a treatment apparatus and a treatment method can be provided which together with being able to treat both hard tissue and soft tissue at a treatment site, allow the obtaining of satisfactory hemostatic effects.

## Claims

1. A treatment apparatus, comprising:
a first light emitting element emitting laser light of a specific wavelength λ₁ within a range of 400 nm to 420 nm;
a first focusing lens which focuses laser light output from the first light emitting element; and
first output means which outputs laser light output from the first light emitting element and focused by the first focusing lens.

2. The treatment apparatus according to claim 1, wherein the first output means is an optic fiber which inputs and guides laser light focused by the first focusing lens to an incident end and outputs the light from an emergent end.

3. The treatment apparatus according to claim 1 or 2, wherein the numerical aperture at the light output end of the first output means is less than 0.14.

4. The treatment apparatus according to any one of claims 1-3 which is a treatment apparatus according to any of claims 1 to 3, comprising: N number of the first light emitting elements S₁ to S_{N} respectively outputting laser light of a specific wavelength λ₁, N number of the first focusing lenses L₁ to L_{N} provided on a 1:1 basis for the N number of light emitting elements S₁ to S_{N} and which focus laser light output from each corresponding first light emitting element, and N number of the optic fibers F₁ to F_{N}, which input and guide laser light output from the N number of first light emitting elements S₁ to S_{N} and focused by the N number of first focusing lenses L₁ to L_{N}, to an incident end, and output the light from an emergent end, wherein the optic fibers Fₙ are bundled at the emergent end (wherein, N represents an integer of 2 or more, and n represents any arbitrary integer from 1 to N).

5. The treatment apparatus according to any one of claims 1-4, comprising a control unit which controls the power of laser light output from the first light emitting elements Sₙ.

6. The treatment apparatus according to claim 5, wherein a non-contact temperature sensor is attached to an emergent end of the optic fiber Fₙ which detects the temperature of a site irradiated with laser light output from that emergent end, and the control unit controls the power of the laser light output from the first light emitting elements Sₙ based on the temperature result detected with the non-contact temperature sensor.

7. The treatment apparatus according to claim 5, wherein the control unit controls the power of the laser light output from the first light emitting elements Sₙ so as to change the irradiation intensity of the laser light output from the first light emitting elements Sₙ over time.

8. The treatment apparatus according to any one of claims 1-7, further comprising:
a second light emitting element which emits laser light of a specific wavelength λ₂ within a range of 800 nm to 1000 nm,
a second focusing lens which focuses laser light output from the second light emitting element, and
second output means which outputs laser light output from the second light emitting element and focused by the second focusing lens.

9. The treatment apparatus according to claim 8, wherein the control unit controls the power of laser light output from the second light emitting element so as to change the irradiation intensity of the laser light output from the second light emitting element over time.

10. The treatment apparatus according to claim 8, wherein the first output means and second output means each radiate laser light so that the respective output laser light is mutually and spatially overlapping.

11. The treatment apparatus according to claim 8, wherein at an irradiated spot formed by the respective laser light from the first output means and the second output means, laser light of a mutually different wavelength at the vicinity of the center of the irradiated spot and at the periphery thereof is radiated by the first output means and the second output means.

12. The treatment apparatus according to claim 11, wherein laser light is irradiated at the periphery of the irradiated spot by the first output means.

13. A treatment method, comprising treating at least hard tissue or soft tissue at a treatment site by irradiating with laser light having a specific wavelength of 400 nm to 420 nm.

14. A treatment method, comprising treating at least hard tissue or soft tissue at a treatment site by irradiating with laser light having a specific wavelength of 400 nm to 420 nm and laser light having a specific wavelength of 800 nm to 1000 nm.

15. The treatment method according to claim 13, wherein the irradiation with the laser light having a specific wavelength of 400 nm to 420 nm is carried out by changing the irradiation intensity over time based on the temperature of the treatment site.

16. The treatment method according to claim 14, wherein the irradiation with at least one of the laser light having a specific wavelength of 400 nm to 420 nm and the laser light having a specific wavelength of 800 nm to 1000 nm is carried out by changing the irradiation intensity over time based on the temperature of the treatment site.
